# EUROPEAN PATENT SPECIFICATION

(11) **EP 3 799 932 B1**
(45) Date of publication and mention of the grant of the patent: **26.10.2022**
(21) Application number: 20202330.5
(22) Date of filing: 10.04.2015
(51) Int. Cl.: A61Q 9/02, A61K 8/02, A61K 8/34, A61K 8/36, A61K 8/46, A61K 8/67, A61K 8/81, A61K 8/85, A61K 8/92, B26B 21/44

(54) **SHAVING AID COMPOSITION**
RASIERHILFSMITTEL
COMPOSITION DE RASAGE

(43) Date of publication of application: 07.04.2021
(62) Divisional of application: 15713949.4
(73) Proprietor: BIC Violex Single Member S.A., 14569 Anoixi (GR)
(72) Inventor: BOUSVAROS, Gerasimos, 145 62 Kifissia (GR); ANTONIOU, Zoi, 190 01 Keratea-attica (GR)
(74) Representative: Peterreins Schley

(56) References cited:
- US-B2- 7 811 553
- Anonymous: "Clariant Industrial & Consumer Specialties Personal Care", , 1 January 2010 (2010-01-01), pages 1-17, XP055777690, Retrieved from the Internet: URL:https://biokhim.com/data2/Clariant/Per sonalCare-Clariant.pdf [retrieved on 2021-02-18]
- DATABASE GNPD [Online] MINTEL; 23 October 2014 (2014-10-23), anonymous: "Perfexion 2 Disposable Razors", XP055777844, Database accession no. 2743627

## Description

### FIELD OF THE INVENTION

The present invention relates to the field of shaving aid compositions. More specifically, the present invention relates to a shaving aid composition intended to be attached to a razor head as a shaving bar for an efficient and simplified shaving.

### BACKGROUND OF THE INVENTION

Conventional razors comprise a lubricating strip including a shaving aid composition which may not be sufficient to provide a comfortable shave by itself (i.e. without the use of an additional shaving aid).

In addition to conventional razors, there are "2 in 1" razors comprising "gel bars" to eliminate need for an additional shaving aid, and razors with a skin conditioning solid which lather while shaving.

However, main problems with existing products involve either too bulky head size or a shaving aid formula which leaves an unpleasant sensation such a "slimy" residual on the skin for example. Also, a high dissolve rate in the shaving aid formula will limit the number of shaves before reaching its full design use life. On the other hand a low dissolve rate will not provide sufficient aid to the user in order to shave adequately without additional shaving aids during each shave.

There is therefore a need in the art for improved shaving aid compositions.

Examples of shaving aid compositions are disclosed in US 7811553 B2, which relates to a shaving aid aid composition comprising a silicone cross-polymer, as soap base containing sodium stearate, semi-solid ester, polyoxyethylene, and from 0.3-10 wt.-% of polyethylene, polybutene and mineral oil composition.

Various cosmetic ingredients, among them polypropylene terephthalate, are disclosed in Clariant brochure "Industrial & Consumer Specialties Personal Care, 2012, published by Clariant Produkte (Deutschland) GmbH.

Incorporation of other functional components within the recommended range into shaving aid composition is thus often desirable for achieving an efficient shave.

A problem that the invention aims to solve is therefore to offer an improved shaving aid composition providing a comfortable, simplified and efficient shaving without the use of an additional shaving aid.

### SUMMARY OF THE INVENTION

For this purpose, an object of the invention is a shaving aid composition comprising:
- a glycol,
- a humectant,
- a solid soap base, and
- a surfactant,
said shaving aid composition further comprising:
- about 0.5 to 4% by weight, preferably 2.5 to 3.5% of a multifunctional polymer wherein the multifunctional polymer is polypropylene terephthalate;

Unless otherwise specified, all percentages and amounts expressed herein and elsewhere in the specification should be understood to refer to percentages by weight calculated with respect to the total weight of the shaving aid composition.

The invention is based in part on the finding that a shaving aid composition comprising notably about 0.5 to 5%, preferably about 0.5-0.8% by weight of an emollient and/or about 0.5 to 4%, preferably 1.5 to 3.5% by weight of a film former and/or about 0.5 to 4% by weight, preferably 2.5 to 3.5% of a multifunctional polymer, can provide excellent shaving properties and skin conditioning benefits.

Indeed, the shaving aid composition according to the invention increases shaving quality and performance, by improving glideness, skin smoothness and skin conditioning.

Furthermore, the composition according to the invention reduces the slimy residue that is known in some existing products and has a right dissolve rate balance.

According to an embodiment, the shaving aid composition comprises about 0.5 to 5%, preferably about 0.5-0.8% by weight of an emollient and about 0.5 to 4%, preferably 1.5 to 3.5% by weight of a film former and about 0.5 to 4% by weight, preferably 2.5 to 3.5% of a multifunctional polymer.

### Emollient

The shaving aid composition according to the invention may include an emollient to bring softness, emolliency and spreadability to the composition.

According to an embodiment; the emollient is hydrogenated polydecene.

According to a particular embodiment; the emollient is hydrogenated polydecene, commercially available as Dekanex ^{®} and is comprised in the shaving aid composition in an amount of 0.5 to 5%, preferably in an amount of 0.5 to 0.8%, and even more preferably in an amount of 0.7% by weight.

### Film former

The shaving aid composition according to the invention may include a film former to improve water resistance and rub resistance.

According to an embodiment, the film former is a mixture comprising hydrogenated polycyclopentadiene, polyethylene, copernica cerifera wax and tocopherol.

According to a particular embodiment, the film former is a mixture comprising hydrogenated polycyclopentadiene, polyethylene, copernica cerifera wax and tocopherol, commercially available as Koboguard HRPC ^{®} and is comprised in an amount of 0.5 to 4%, preferably in an amount of 1.5 to 3.5%, and even more preferably in an amount of 2% by weight.

### Multifunctional polymer

The shaving aid composition according to the invention includes a multifunctional polymer to improve skin feel and foam aesthetics.

The multifunctional polymer is polypropylene terephthalate.

The multifunctional polymer is selected from the group consisting of polypropylene terephthalate, commercially available as Aristoflex PEA ^{®} and is comprised in an amount of 0.5 to 4%, preferably in an amount of 2.5 to 3.5%, and even more preferably in an amount of 3% by weight.

### Glycol

According to the invention, the composition comprises a glycol which acts as a viscosity adjuster and/or as a solvent and/or as a skin conditioning agent.

According to an embodiment, the glycol is chosen in the group consisting of propylene glycol, ethylene glycol, diethylene glycol and mixtures thereof.

According to a particular embodiment, the composition comprises propylene glycol.

According to an embodiment, the composition comprises a glycol in an amount of 20-35% by weight, preferably in an amount of 24-30% by weight.

### Humectant

The shaving aid composition according to the invention includes a humectant to impart hydrating properties.

Humectant(s) may be used in an amount of about 30-50% by weight, preferably, in an amount of 33-38% by weight.

Humectants useful in the present invention are those generally known in the art and include polyhydric alcohols selected from the group consisting of glycerin, sorbitol and mixture thereof.

According to an embodiment, the shaving aid composition comprises a mixture of sorbitol and glycerin.

Humectants useful in the present invention can also include other components such as Aloe Vera powder, for example in an amount of 0.001-0.05% by weight and/or shea butter for example in an amount of 0.2-1% by weight.

According to a particular embodiment, the composition comprises a mixture of 15-25% by weight of sorbitol, preferably 15-18% by weight of sorbitol and 15-25% by weight of glycerin, preferably 16-20% by weight of glycerin. The high amount of glycerin gives notably comfort to the skin.

### Soap base

A variety of solid soap bases as those which are well known in the art may be employed in the composition according to the invention.

The soap base useful in the present invention includes fatty acid ester in C₁₀-C₂₄, preferably in C₁₄-C₂₄ such as sodium and/or potassium cocoate, stearate, palmitate, myristate and mixtures thereof.

A particularly preferred soap base is a sodium stearate soap base.

According to an embodiment, the soap base is used in an amount of 15-24% by weight, preferably in an amount of 18-22% by weight.

### Surfactant

Surfactant(s) used in the present invention provide cleansing and emulsifying properties.

Surfactant(s) useful in the present invention are those generally known in the art. According to an embodiment, the surfactant is an anionic surfactant, preferably sodium laureth sulfate.

According to an embodiment, the surfactant is used in an amount of 6.5-12.5% by weight, preferably in an amount of 8-12% by weight.

### Other components

According to an embodiment, the shaving aid composition contains at least one additional component that is added to impart specific properties such as coloring agent, fragrance, chelating agent, opacifier, antibacterial agent or exfoliating particles.

The coloring agents are used in very low range, typically used in range from about 0.0001% to about 0.001% by weight.

Fragrance is also used in very low range, preferably no more than 0.5% by weight.

When the composition comprises a chelating agent, it is included in a range from 0.05% to 0.2% by weight. Different chelating agent, well known in the art in the cosmetic field, may be employed in the composition according to the invention, as for example Dissolvine GL47S ^{®}

When the composition comprises an opacifier, it is included in a range from 0.2% to 2% by weight. Different opacifier, well-known in the art in the cosmetic field, may be employed in the composition according to the invention, as for example Opulyn 301 ^{®}.

According to a particular embodiment, the shaving aid composition of the present invention comprises:
- about 20-35% by weight, preferably 24-30% by weight of a glycol;
- about 30-50% by weight, preferably 33-38% by weight of a humectant
- about 15-24% by weight, preferably 18-22% by weight of a soap base,
- about 6.5-12.5% by weight, preferably 8-12% by weight of a surfactant,
- about 0.5-5% by weight, preferably 0.5-0.8% of hydrogenated polydecene,
- about 0.5-4% by weight, preferably 2.5-3.5% of polypropylene terephthalate; and
- about 0.5-4% by weight, preferably 1.5-3.5% of a mixture comprising hydrogenated polycyclopentadiene, polyethylene, copernica cerifera wax and tocopherol.

According to another particular embodiment, the shaving aid composition of the present invention comprises:
- about 20-35% by weight, preferably 24-30% by weight of propylene glycol;
- about 15-25% by weight, preferably 16-20% by weight of glycerin,
- about 15-25% by weight, preferably 15-18% by weight of sorbitol,
- about 15-24% by weight, preferably 18-22% by weight of sodium stearate,
- about 6.5-12.5% by weight, preferably 8-12% by weight of sodium laureth sulfate,
- about 0.5-5% by weight, preferably 0.5-0.8% of hydrogenated polydecene,
- about 0.5-4% by weight, preferably 2.5-3.5% of polypropylene terephthalate; and
- about 0.5-4% by weight, preferably 1.5-3.5% of a mixture comprising hydrogenated polycyclopentadiene, polyethylene, copernica cerifera wax and tocopherol.

The shaving aid composition according to the invention can be manufactured by the following method comprising the step consisting of:
(i) dissolving a film former in a first amount of glycol to form a first mixture;
(ii) preparing a second mixture comprising a second amount of glycol with a humectant;
(iii) adding the first mixture to the second mixture;
(iv) adding a surfactant;
(v) adding an emollient and a multifunctional polymer to the mixture obtained in step (iv); and
(vi) optionally adding additional components chosen in the group consisting of coloring agent, fragrance, chelating agent, opacifier, antibacterial agent or exfoliating particles to the mixture obtained in step (v).

Then, a shaving bar can be prepared from the shaving aid composition by process, well-known from the person skilled in the art, i.e by extrusion or by molding.

Another object of the present invention is a razor cartridge comprising:
- a housing having a front edge and a rear edge;
- one or more shaving blades between the front edge and the rear edge; and
- at least one shaving bar disposed in front of the blade(s) and/or at rear of the blade(s), the shaving bar being made of the shaving aid composition as defined previously.

According to an embodiment, the shaving bar is disposed in a shaving bar holder.

According to a particular embodiment, the razor cartridge comprises two shaving bars, one disposed in front of the blade(s) and another disposed at rear of the blade(s).

According to another embodiment, the razor cartridge comprises an additional strip, such as a lubricating strip disposed between the blade(s) and the shaving bar.

Another object of the present invention is a razor comprising:
- a handle, and
- a razor cartridge as defined previously.

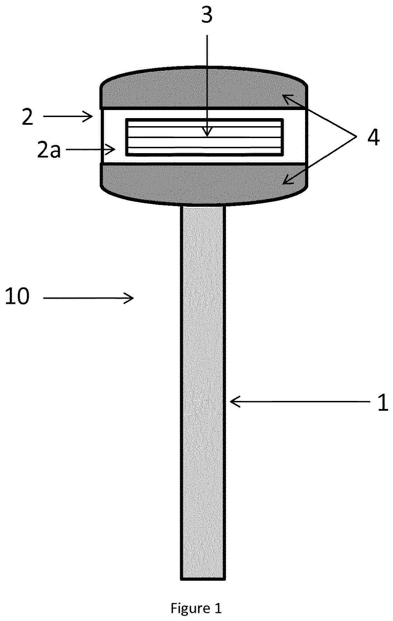

Figure 1 represents a razor including a razor cartridge, said razor cartridge comprising a shaving bar made of the shaving aid composition of the present invention.

The razor 10 of figure 1 comprises a handle 1 and a razor cartridge 2 having a housing 2a, said housing 2a including a front edge and a rear edge (not represented in figure 1).

Several blades 3 are disposed between the front edge and the rear edge of the housing 2a.

In this embodiment, a shaving bar 4 is disposed in front of the blades 3 and another shaving bar 4 is disposed at rear of the blades 3, the shaving bars 4 being made of shaving aid composition of the present invention.

When the shaving bar is in contact with water, foam is formed in contact with the skin providing an efficient and pleasant shaving without the need of an additional shaving aid.

The use of a mixture comprising about 0.5 to 5% by weight of an emollient, and/or about 0.5 to 4% of a film former; and/or about 0.5 to 4% of a multifunctional polymer in a shaving aid composition provides softness, emolliency and spreadability.

### EXAMPLE 1

The composition of the present invention was prepared by a hot mix of the components described in the table below.

**Table 1: shaving aid composition of example 1**

| **Components** | **Amount (%)w** |
|---|---|
| Propylene glycol | 20%-35% |
| Glycerin | 15%-25% |
| Sodium stearate | 15%-24% |
| Sorbitol | 15%-25% |
| Sodium Polyoxyethylene Laurylether Sulfate | 6.5%-12.5% |
| Hydrogenated Polycyclopentadiene | 1.6 |
| Polyethylene | |
| Copernicia Cerifera (Carnauba) Wax | |
| Tocopherol | |
| hydrogenated polydecene | 0,5 |
| Polypropylene Terephthalate | 2.5 |
| Opulyn 301 ^{®} | 0.2%-2% |
| Shea butter | 0.2%-1% |
| Aloe vera powder | 0.001%-0.05% |
| Perfume | <0.5% |
| Dissolvine GL47S ^{®} | 0.05%-0.2% |
| Color | 0.0001%-0.001% |

where
Opulyn ^{®} 301 composition: Styrene/acrylic copolymer, Residual monomers, water.

## Claims

1. A shaving aid composition comprising:
a glycol, a humectant,
a solid soap base, and
a surfactant,
**characterized in that** it further comprises:
about 0.5 to 4% by weight, preferably 2.5 to 3.5% of a multifunctional polymer wherein the multifunctional polymer is polypropylene terephthalate;
said percentages being calculated with respect to the total weight of the shaving aid composition.

2. The shaving aid composition according to claim 1, wherein the composition further comprises an emollient.

3. The shaving aid composition according to claim 2, wherein the emollient is hydrogenated polydecene.

4. The shaving aid composition according to any one of the preceding claims, wherein the composition further comprises a film former.

5. The shaving aid composition according to claim 4, wherein the film former is a mixture comprising hydrogenated polycyclopentadiene, polyethylene, copernica cerifera wax and tocopherol.

6. The shaving aid composition according to any one of the preceding claims, wherein the composition comprises a glycol in an amount of 20-35% by weight.

7. The shaving aid composition according to claim 5, wherein the glycol is propylene glycol.

8. The shaving aid composition according to any one of the preceding claims, wherein the humectant is chosen in the group consisting of glycerin, sorbitol and mixtures thereof.

9. The shaving aid composition according to any one of the preceding claims, wherein the soap base is chosen in the group consisting of fatty acid ester in C10-C24, more specifically wherein the soap base is sodium and/or potassium cocoate, stearate, palmitate, myristate and mixtures thereof, more specifically sodium stearate.

10. The shaving aid composition according to any one of the preceding claims, wherein the surfactant is sodium laureth sulfate.

11. The shaving aid composition according to any one of the preceding claims comprising:
about 20-35% by weight, preferably 24-30% by weight of propylene glycol;
about 15-25% by weight, preferably 16-20% by weight of glycerin,
about 15-25% by weight, preferably 15-18% by weight of sorbitol,
about 15-24% by weight, preferably 18-22% by weight of sodium stearate,
about 6.5-12.5% by weight, preferably 8-12% by weight of sodium laureth sulfate,
about 0.5-5% by weight, preferably 0.5-0.8% of hydrogenated polydecene,
about 0.5-4% by weight, preferably 2.5-3.5% of polypropylene terephthalate;
about 0.5-4% by weight, preferably 1.5-3.5% of a mixture comprising hydrogenated polycyclopentadiene, polyethylene, copernica cerifera wax and tocopherol, said percentages being calculated with respect to the total weight of the shaving aid composition.

12. A razor cartridge (2) comprising:
a housing (2a) having a front edge and a rear edge;
one or more shaving blades (3) between the front edge and
the rear edge; and
at least one shaving bar (4) disposed in front of the blade(s) and/or at rear of the blade(s), the shaving bar (4) being made of the shaving aid composition according to any one of claims 1-11.

## Patentansprüche

1. Rasiermittelzusammensetzung, umfassend:
ein Glykol,
ein Feuchthaltemittel,
eine feste Seifenbasis und
ein Tensid,
**dadurch gekennzeichnet, dass** sie ferner umfasst:
etwa zu 0,5 bis 4 Gew.-%, vorzugsweise zu 2,5 bis 3,5 Gew.-%, ein multifunktionelles Polymer, wobei das multifunktionelle Polymer Polypropylenterephthalat ist;
wobei die Prozentsätze hinsichtlich des Gesamtgewichts der Rasiermittelzusammensetzung berechnet werden.

2. Rasiermittelzusammensetzung nach Anspruch 1, wobei die Zusammensetzung ferner einen Weichmacher umfasst.

3. Rasiermittelzusammensetzung nach Anspruch 2, wobei der Weichmacher hydriertes Polydecen ist.

4. Rasiermittelzusammensetzung nach einem der vorstehenden Ansprüche, wobei die Zusammensetzung ferner einen Filmbildner umfasst.

5. Rasiermittelzusammensetzung nach Anspruch 4, wobei der Filmbildner eine Mischung, umfassend hydriertes Polycyclopentadien, Polyethylen, Carnaubawachs und Tocopherol ist.

6. Rasiermittelzusammensetzung nach einem der vorstehenden Ansprüche, wobei die Zusammensetzung ein Glykol in einer Menge von 20-35 Gew.-% umfasst.

7. Rasiermittelzusammensetzung nach Anspruch 5, wobei das Glykol Propylenglykol ist.

8. Rasiermittelzusammensetzung nach einem der vorstehenden Ansprüche, wobei das Feuchthaltemittel aus der Gruppe ausgewählt ist, bestehend aus Glyzerol, Sorbit und Mischungen davon.

9. Rasiermittelzusammensetzung nach einem der vorstehenden Ansprüche, wobei die Seifenbasis aus der Gruppe ausgewählt ist, bestehend aus Fettsäureester in C10-C24, noch spezifischer, wobei die Seifenbasis Natrium- und/oder Kaliumcocoat, -stearat, -palmitat, -myristat und Mischungen davon, noch spezifischer Natriumstearat, ist.

10. Rasiermittelzusammensetzung nach einem der vorstehenden Ansprüche, wobei das Tensid Natriumlaurethsulfat ist.

11. Rasiermittelzusammensetzung nach einem der vorstehenden Ansprüche, umfassend:
zu etwa 20-35 Gew.-%, vorzugsweise zu 24-30 Gew.-% Propylenglykol;
zu etwa 15-25 Gew.-%, vorzugsweise zu 16-20 Gew.-% Glyzerol,
zu etwa 15-25 Gew.-%, vorzugsweise zu 15-18 Gew.-% Sorbit,
zu etwa 15-24 Gew.-%, vorzugsweise zu 18-22 Gew.-% Natriumstearat,
zu etwa 6,5-12,5 Gew.-%, vorzugsweise zu 8-12 Gew.-% Natriumlaurethsulfat,
zu etwa 0,5-5 Gew.-%, vorzugsweise zu 0,5-0,8 Gew.-% hydriertes Polydecen,
zu etwa 0,5-4 Gew.-%, vorzugsweise zu 2,5-3,5 Gew.-% Polypropylenterephthalat;
zu etwa 0,5-4 Gew.-%, vorzugsweise zu 1,5-3,5 Gew.-% eine Mischung, umfassend hydriertes Polycyclopentadien,
Polyethylen, Carnaubawachs und Tocopherol, wobei die Prozentsätze hinsichtlich des Gesamtgewichts der Rasiermittelzusammensetzung berechnet werden.

12. Rasiererkassette (2), umfassend:
ein Gehäuse (2a), das eine Vorderkante und eine Hinterkante aufweist;
eine oder mehrere Rasierklingen (3) zwischen der Vorderkante und der Hinterkante; und
mindestens einen Rasiergleitstreifen (4), der vor der/den Klinge(n) und/oder hinter der/den Klinge(n) angeordnet ist, wobei der Rasiergleitstreifen (4) aus der Rasiermittelzusammensetzung nach einem der Ansprüche 1 bis 11 hergestellt ist.

## Revendications

1. Composition d'aide au rasage comprenant :
un glycol,
un humectant,
une base de savon solide, et
un tensioactif,
**caractérisée en ce qu'**elle comprend en outre :
environ 0,5 à 4 % en poids, de préférence 2,5 à 3,5 % d'un polymère multifonctionnel, dans laquelle le polymère multifonctionnel est le poly(téréphtalate de propylène) ;
lesdits pourcentages étant calculés par rapport au poids total de la composition d'aide au rasage.

2. Composition d'aide au rasage selon la revendication 1, dans laquelle la composition comprend en outre un émollient.

3. Composition d'aide au rasage selon la revendication 2, dans laquelle l'émollient est du polydécène hydrogéné.

4. Composition d'aide au rasage selon l'une quelconque des revendications précédentes, dans laquelle la composition comprend en outre une substance filmogène.

5. Composition d'aide au rasage selon la revendication 4, dans laquelle la substance filmogène est un mélange comprenant du polycyclopentadiène hydrogéné, du polyéthylène, de la cire de copernica cerifera et du tocophérol.

6. Composition d'aide au rasage selon l'une quelconque des revendications précédentes, dans laquelle la composition comprend un glycol en une quantité de 20 à 35 % en poids.

7. Composition d'aide au rasage selon la revendication 5, dans laquelle le glycol est le propylèneglycol.

8. Composition d'aide au rasage selon l'une quelconque des revendications précédentes, dans laquelle l'humectant est choisi dans le groupe constitué par le glycérol, le sorbitol et leurs mélanges.

9. Composition d'aide au rasage selon l'une quelconque des revendications précédentes, dans laquelle la base de savon est choisie dans le groupe constitué par un ester d'acide gras en C10-C24, plus particulièrement dans laquelle la base de savon est le cocoate, stéarate, palmitate, myristate de sodium et/ou de potassium, et leurs mélanges, plus particulièrement le stéarate de sodium.

10. Composition d'aide au rasage selon l'une quelconque des revendications précédentes, dans laquelle le tensioactif est le laurethsulfate de sodium.

11. Composition d'aide au rasage selon l'une quelconque des revendications précédentes, comprenant :
environ 20 à 35 % en poids, de préférence 24 à 30 % en poids de propylèneglycol ;
environ 15 à 25 % en poids, de préférence 16 à 20 % en poids de glycérol,
environ 15 à 25 % en poids, de préférence 15 à 18 % en poids de sorbitol,
environ 15 à 24 % en poids, de préférence 18 à 22 % en poids de stéarate de sodium,
environ 6,5 à 12,5 % en poids, de préférence 8 à 12 % en poids de laurethsulfate de sodium,
environ 0,5 à 5 % en poids, de préférence 0,5 à 0,8 % de polydécène hydrogéné,
environ 0,5 à 4 % en poids, de préférence 2,5 à 3,5 % de poly(téréphtalate de propylène) ;
environ 0,5 à 4 % en poids, de préférence 1,5 à 3,5 % d'un mélange comprenant du polycyclopentadiène hydrogéné, du polyéthylène, de la cire de copernica cerifera et du tocophérol, lesdits pourcentages étant calculés par rapport au poids total de la composition d'aide au rasage.

12. Cartouche de rasoir (2) comprenant :
un boîtier (2a) présentant un bord avant et un bord arrière ;
une ou plusieurs lames de rasage (3) entre le bord avant et le bord arrière ; et
au moins une bande de rasage (4) disposée devant la ou les lames et/ou à l'arrière de la ou des lames, la bande de rasage (4) étant constituée de la composition d'aide au rasage selon l'une quelconque des revendications 1 à 11.
